(19) Europäisches Patentamt
European Patent Office
Office européen des brevets

(11) **EP 4 366 682 B1**

(12) **EUROPEAN PATENT SPECIFICATION**

(45) Date of publication and mention
of the grant of the patent:
**29.01.2025 Bulletin 2025/05**

(21) Application number: **22744160.7**

(22) Date of filing: **04.07.2022**

(51) International Patent Classification (IPC):
*A61K 8/28* (2006.01)     *A61Q 15/00* (2006.01)
*A61K 8/44* (2006.01)     *A61K 8/20* (2006.01)

(52) Cooperative Patent Classification (CPC):
**A61K 8/28; A61K 8/20; A61K 8/44; A61Q 15/00;**
A61K 2800/58; A61K 2800/805

(86) International application number:
**PCT/EP2022/068405**

(87) International publication number:
**WO 2023/280755 (12.01.2023 Gazette 2023/02)**

(54) **METHOD OF MANUFACTURE OF ANTIPERSPIRANT SALTS**

VERFAHREN ZUR HERSTELLUNG VON TRANSPIRATIONSHEMMENDEN SALZEN

PROCÉDÉ DE FABRICATION DE SELS ANTISUDORIFIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.07.2021 EP 21184287**

(43) Date of publication of application:
**15.05.2024 Bulletin 2024/20**

(73) Proprietors:
• **Unilever IP Holdings B.V.**
**3013 AL Rotterdam (NL)**
Designated Contracting States:
**AL AT BE BG CH CZ DK EE ES FI FR GR HR HU IS
LI LT LU LV MC MK NL NO PL PT RO SE SI SK SM**

• **Unilever Global IP Limited**
**Wirral, Merseyside CH62 4ZD (GB)**
Designated Contracting States:
**CY DE GB IE IT MT RS TR**

(72) Inventors:
• **KAY, Cameron**
**6708 WH Wageningen (NL)**
• **LUCKWELL, Craig James**
**6708 WH Wageningen (NL)**

(74) Representative: **Whaley, Christopher**
**Unilever Patent Group**
**Bronland 14**
**6708 WH Wageningen (NL)**

(56) References cited:
GB-A- 2 091 099          US-A- 5 955 065
US-A1- 2007 003 499      US-B1- 6 245 325

## Description

### Field of Invention

The present invention is concerned with a method of manufacture of antiperspirant (AP) salts, particularly aluminium and zirconium containing AP salts having enhanced activity.

### Background

Methods for the manufacture aluminium-containing AP salts having enhanced activity are well known in the prior art. The AP salts produced are often described as "activated".

Traditionally, activated AP salts have been prepared by prolonged heating of basic aluminium chloride solutions followed by spray-drying; see, for example, US 4,359,456 (Gosling). The samples prepared by this method needed to be formulated into essentially anhydrous compositions in order for the AP salt to maintain its high activity.

Activated AP salts have also been prepared using water-soluble calcium acids, particularly with a further adjunct such as an amino acid, hydroxyl acid, or betaine. Some of these samples could be formulated into aqueous compositions without the AP salt losing all of its enhanced activity.

EP 1,104,282 (Gillette) discloses a means of producing activated AP salts using a water-soluble calcium salt and glycine or a hydroxy acid.

US 6,911,195 (Gillette) discloses water-in-oil emulsion gels comprising aluminium-zirconium AP salts activated using calcium ions.

US 5,955,065 (Gillette) discloses anhydrous suspension formulations comprising particulate AP salts and aluminium-zirconium AP salts activated using calcium ions.

US 6,942,850 (Gillette) discloses aqueous alcoholic composition comprising aluminium-zirconium AP salts activated using calcium ions.

WO 2009/044381 (P&G) discloses water-in-oil emulsion sticks comprising aluminium and aluminium-zirconium AP salts activated using calcium ions.

US 7,704,531 (Colgate) discloses compositions comprising an active system made from combining an aluminium or aluminium-zirconium salt, a calcium salt, and a betaine.

US 2011/0038823 (Dial/Henkel) discloses water-in-oil emulsion sticks comprising an AP active prepared by combining BAC, calcium chloride and glycine.

US 2007/196303, US 2007/0020211, WO 2008/063188, US 2008/0131354 and US 7,087,220 (Summit and Reheis) each describe methods of making calcium-activated AP salts.

US 2007/0003499 (P&G) discloses enhanced efficacy aluminium-zirconium chlorohydrex acid complexes in combination with a neutralizing salt, the AP salt having a pH at 15% by weight of greater than 5.

### Summary of Invention

The present invention is concerned with the manufacture of AP salts of enhanced activity and compositions comprising such salts. The salts must be aluminium-zirconium salts. The activation method involves the use of a water-soluble calcium salt and glycine and takes place in aqueous solution at elevated temperature.

It is an object of the present invention to provide a manufacturing route to AP actives having superior efficacy.

It is a further object of the present invention to provide a manufacturing route to AP actives that can be formulated into AP compositions having low irritation potential.

It is a further object of the present invention to provide a manufacturing route to AP actives that can be formulated into AP compositions having good rheological stability.

In a first aspect of the present invention, there is provided a method of manufacture of an aluminium-zirconium (Al/Zr) antiperspirant active comprising the heating in aqueous solution of an aluminium-zirconium tri- tetra- or penta-chlorohydrex-glycine complex and a water-soluble calcium salt, the molar ratio of aluminium to calcium being from 1.3 to 60.

In a second aspect of the present invention, there is provided an AP composition comprising an AP active manufactured by a method according to the first aspect of the present invention.

In a third aspect of the present invention, there is provided a cosmetic method of obtaining an antiperspirancy benefit comprising the topical application to the surface of the human body of an AP active manufactured by a method according to the first aspect of the present invention.

Detailed Description

Herein, features expressed as "preferred" with regard to a particular aspect of the invention should be understood to be preferred with regard to each aspect of the invention (likewise, features expressed as "more preferred", "particularly preferred" or "most preferred"). Preferred" features aid the delivery of one or more of the objects of the invention.

Herein, preferred features of the invention are particularly preferred when used in combination with other preferred features (likewise, features expressed as "more preferred", "particularly preferred" or "most preferred"). "

Herein, "ambient conditions" refer to 20°C and 1 atmosphere pressure, unless otherwise indicated.

Herein, all percentages, ratios and amounts are by weight, unless otherwise indicated. Herein, amounts and concentrations of ingredients are percentages by weight of the total composition, unless otherwise indicated.

Herein, ratios expressed as, for example, "Al: Ca is from 1.3 to 60", means that Al: Ca is from 1.3: 1 to 60: 1.

Herein, references to molar amounts and ratios of "aluminium" are calculated on the basis of mono-nuclear aluminium, but include aluminium present in poly-nuclear species; indeed, most of the aluminium in the salts of relevance is present in poly-nuclear species.

Herein, references to amounts of components such as "carrier oil" or "thickening agent" relate to the total amount of such components present in the composition.

Herein, the word "comprising" is intended to mean "including" but not necessarily "consisting of', i.e., it is non-exhaustive.

Herein, "cosmetic" methods and compositions should be understood to mean non-therapeutic methods and compositions, respectively.

Herein the term "AP salt" may be used to refer to the aluminium-zirconium-glycine complex used in the present invention.

The method of the present invention leads to AP compositions delivering surprisingly good antiperspirancy performance. In addition, such compositions have good storage stability, in particular rheological stability. Further, such compositions have low irritancy due to their relatively high pH.

The AP salt produced by the present invention is manufactured from an aluminium-zirconium tri- tetra- or penta-chlorohydrex-glycine complex, "activated" with a water-soluble calcium salt (*vide infra*).

Herein, AP salts referred to as "octa salts", including those complexed with glycine, are aluminium-zirconium octa-chlorohydrex species wherein the aluminium to zirconium ratio is from 6 to 10 and the metal to chloride ratio is from 0.9 to 1.5. That is to say:

$$Al: Zr \quad = \quad 6: 1 \text{ to } 10: 1$$

and

$$(Al + Zr): Cl \quad = \quad 0.9: 1 \text{ to } 1.5: 1.$$

An example of such a salt is $Al_8Zr(OH)_{20}Cl_8$, wherein Al: Zr is 8 and (Al + Zr): Cl is 1.125.

Herein, AP salts referred to as "penta salts", including those complexed with glycine, are aluminium-zirconium penta-chlorohydrex species wherein the aluminium to zirconium ratio is from 6 to 10 and the metal to chloride ratio is from 1.51 to 2.1. That is to say:

$$Al: Zr \quad = \quad 6: 1 \text{ to } 10: 1$$

and

$$(Al + Zr): Cl \quad = \quad 1.51: 1 \text{ to } 2.1: 1.$$

An example of such a salt is $Al_8Zr(OH)_{23}Cl_5$, wherein Al: Zr is 8 and (Al + Zr): Cl is 1.8.

Penta salts in accordance with the invention are complexed with glycine.

Herein, AP salts referred to as "tetra salts", including those complexed with glycine, are aluminium-zirconium tetra-chlorohydrex species wherein the aluminium to zirconium ratio is from 2 to 5.99 and the metal to chloride ratio is from 0.9 to 1.5. That is to say:

$$Al: Zr \quad = \quad 2: 1 \text{ to } 5.99: 1$$

and

$$(\text{Al} + \text{Zr}) : \text{Cl} \qquad = \qquad 0.9 : 1 \text{ to } 1.5 : 1.$$

An example of such a salt is $Al_4Zr(OH)_{12}Cl_4$, wherein Al: Zr is 4 and (Al + Zr): Cl is 1.25.

Tetra salts according to the invention are complexed with glycine.

Herein, AP salts referred to as "tri salts", including those complexed with glycine, are aluminium-zirconium tri-chlorohydrex species wherein the aluminium to zirconium ratio is from 2 to 5.99 and the metal to chloride ratio is from 1.51 to 2.1. That is to say:

$$\text{Al} : \text{Zr} \qquad = \qquad 2 : 1 \text{ to } 5.99 : 1$$

and

$$(\text{Al} + \text{Zr}) : \text{Cl} \qquad = \qquad 1.51 : 1 \text{ to } 2 : 1.$$

An example of such a salt is $Al_4Zr(OH)_{13}Cl_3$, wherein Al: Zr is 4 and (Al + Zr): Cl is 1.667.

Tri salts according to the invention are complexed with glycine.

The AP salt produced by the present invention is preferably an aluminium-zirconium penta-chlorohydrex-glycine complex and is produced from an aluminium-zirconium penta-chlorohydrex salt, including the possibility that the penta-chlorohydrex-glycine complex is produced *in situ* during the heating in aqueous solution of a penta-chlorohydrex salt, glycine and a water-soluble calcium salt.

During the manufacturing method of the invention, the concentration of the aluminium-zirconium tri- tetra- or penta-chlorohydrex-glycine complex is preferably from 20 to 60%, more preferably from 25 to 50% and most preferred from 30 to 45%, including all of the glycine present, whether present in an Al-Zr complex added or independently added.

The method manufacture of the invention produces a mixture of aluminium-zirconium species having a relatively high content of what is commonly termed Band 3 material, as determined by SEC (Size Exclusion Chromatography). The SEC technique employed is well known in the art and is described in further detail in US 4,359,456 (Gosling). The SEC band commonly referred to as Band 3 is designated as "Peak 4" in EP 1,104,282 B1 by Gillette. Having a high content of Band 3 material has been associated with high antiperspirancy performance in the prior art.

Herein, "Band 3 content" refers to the integrated area in the Band 3 region of the SEC chromatograph relative to the total integrated area in all of the regions corresponding to aluminium species; that is to say, Bands 1, 2, 3, and 4.

The Band 3 content of compositions produced according to the invention is preferably at least 30%, more preferably at least 50% and most preferably at least 70%.

In the method of manufacture described herein, it is preferred that the mixture is heated for sufficient time and at sufficient temperature for the Band 3 content of the aluminium-zirconium species to become at least 30%, more preferably at least 50% and most preferably at least 70%.

The Band 3: Band 2 ratio is a further criterion by which the extent of activation of the Al/Zr AP salt may be judged. The Band 3: Band 2 ratio is the integrated area in the Band 3 region of the SEC chromatograph divided by the integrated area in the Band 2 region. The Band 3 to Band 2 ratio in the actives resulting from the manufacturing method of the invention is preferable at least 3, for example from 3 to 10, and more preferably at least 5, for example from 5 to 10.

Preferred water-soluble calcium salts used in accordance with the invention have a solubility in water of 10 g/L or greater, e.g. 10-100 g/L, under ambient conditions. A particularly preferred water-soluble calcium salt is calcium chloride.

The content of water-soluble calcium salt in the method of manufacture is preferably from 3 to 20%, more preferably from 4 to 15% and most preferred from 6 to 12.5%.

The level of water-soluble calcium salt used in the invention is such that the molar ratio of aluminium to calcium is from 1.3 to 60 and preferably from 1.8 to 20. For aluminium-zirconium penta-chlorohydrex-glycine complexes, the molar ratio of aluminium to calcium is preferably from 1.5 to 60, more preferably from 1.8 to 20, still more preferably from 2 to 10 and most preferably from 2 to 7.

The glycine used in the present invention may be used *per se* or as a salt thereof, such glycine hydrochloride. It may also be used as a pre-formed complex with an aluminium-zirconium tri- tetra- or penta-chlorohydrex salt. Amounts and ratios relating to glycine are to the total glycine content *per se.*

The total content of glycine in the method of manufacture is preferably from 1 to 15% and more preferably from 2 to 12%. For aluminium-zirconium penta-chlorohydrex-glycine complexes, the total content of glycine is preferably from 1 to 15%, more preferably from 2 to 12% and most preferably from 3 to 10%.

The total level of glycine used in the invention is such that the molar ratio of aluminium to glycine is preferably from 1 to 25 and more preferably from 1.5 to 15. For aluminium-zirconium penta-chlorohydrex-glycine complexes, the molar ratio of aluminium to glycine is preferably from 1.5 to 20, more preferably from 2 to 10 and most preferably from 2 to 6.

The method of manufacture involves the heating together in aqueous solution of an aluminium-zirconium tri- tetra- or penta-chlorohydrex-glycine complex and a water-soluble calcium salt. In some embodiments, the aluminium-zirconium tri- tetra- or penta-chlorohydrex-glycine complex is produced *in situ* from an aluminium-zirconium tri- tetra- or penta-chlorohydrex salt and glycine, by heating in aqueous solution with the water-soluble calcium salt.

The heating together of the components in aqueous solution causes "activation" of the antiperspirant salt, increasing its Band 3 content and its Band 3: Band 2 ratio (*vide infra*). This higher Band 3 content leads to a more effective antiperspirant active, as previously recognised in the literature.

In preferred embodiments, the method of manufacture involves heating the components together for 30 minutes or longer at a temperature of 50°C or above. In particularly preferred embodiments, the method involves heating the components together for 60 minutes or longer at a temperature of 70°C or above, for example 1 to 2 hours at 70°C to 90°C.

Following this heat activation step, the aqueous solution is typically allowed to cool to ambient temperature. The solution may then be used in the immediate preparation of antiperspirant compositions or it may be stored before being used. Aqueous antiperspirant salt solutions produced according to the invention have been found to have good storage stability, particularly the Tetra- and Penta- salts (*vide infra*).

The aqueous antiperspirant salt solution produced according to the invention may be used "as is" or it may be diluted or concentrated prior to use. One form of "concentration" involves spray drying of the aqueous antiperspirant salt solution to give a particulate solid. This is typically milled and may be used in a range of antiperspirant compositions, such as suspension sticks and soft solids.

AP actives prepared in accordance with the invention may be incorporated into AP compositions which are to be considered AP compositions according to the invention.

AP compositions according to the invention are preferably aqueous compositions, but they may also be anhydrous suspension sticks or anhydrous soft solids. Indeed, a further aspect of the invention is an anhydrous AP stick compositions or anhydrous AP soft solid composition wherein the AP active is prepared in accordance with the first aspect of the invention.

The pH of the aqueous solution used in the present invention is preferably from 3.5 to 4.1 and more preferably from 3.6 to 4.0. These are relatively high pH ranges for AP solutions and can assist with reducing the irritancy potential of AP compositions prepared in accordance with the invention.

A preferred additional component of compositions of the invention is an oil. Herein, the terms "oil" and signifies a water-insoluble organic material that is liquid at 20°C. Any material having a solubility of less than 0.1g/100g at 20°C is considered to be insoluble.

A preferred oil for use in compositions prepared in accordance with the present invention is a fragrance oil, sometimes alternatively called a perfume oil. The fragrance oil may comprise a single fragrance or component more commonly a plurality of fragrance components. Herein, fragrance oils impart an odour, preferably a pleasant odour, to the composition. Preferably, the fragrance oil imparts a pleasant odour to the surface of the human body the composition is applied to the same.

The amount of fragrance oil in the composition is commonly up to 3% advantageously is at least 0.5% and particularly from 0.8% to 2%.

The total amount of oil in the composition is preferably from 0.1 to 20%, more preferably from 0.5 to 10%, and most preferably at from 2 to 8% by weight of the total composition. In certain preferred embodiments, particularly those also comprising an aluminium and/or zirconium containing AP active, the oil is present at greater than 2.5% and less than 6% by weight of the total composition.

In certain embodiments, it is preferred to include an oil, other than a fragrance oil, that has a relatively low viscosity, by which is meant less 250 cS ($mm^2.s^{-1}$). Such oils can improve the sensory properties of the composition on application and can lead to other benefits such as emolliency.

Suitable oils can be selected from alkyl ether oils having a boiling point of above 100°C and especially above 150°C, including polyalkyleneglycol alkyl ethers. Such ethers desirably comprise between 10 and 20 ethylene glycol or propylene glycol units and the alkyl group commonly contains from 4 to 20 carbon atoms. The preferred ether oils include polypropylene glycol alkyl ethers such as PPG-14-butylether and PPG-15-stearyl ether.

Suitable oils can include one or more triglyceride oils. The triglyceride oils commonly comprise the alkyl residues of aliphatic $C_7$ to $C_{20}$ alcohols, the total number of carbon atoms being selected in conjunction with the extent of olefinic unsaturation and/or branching to enable the triglyceride to be liquid at 20°C. One example is jojoba oil. Particularly preferably, in the triglyceride oil the alkyl residues are linear $C_{18}$ groups having one, two or three olefinic degrees of unsaturation, two or three being optionally conjugated, many of which are extractable from plants (or their synthetic analogues), including triglycerides of oleic acid, linoleic acid, conjugated linoleic acids, linolenic acid, petroselenic acid, ricinoleic acid, linolenelaidic acid, trans 7-octadecenoic acid, parinaric acid, pinolenic acid, punicic acid, petroselenic acid and stearidonic acid.

Suitable oils can include those derived from unsaturated $C_{18}$ acids, including coriander seed oil, impatiens balsimina seed oil, parinarium laurinarium kernel fat oil, sabastiana brasilinensis seed oil, dehydrated castor seed oil, borage seed oil, evening primrose oil, aquilegia vulgaris oil, sunflower (seed) oil and safflower oil. Other suitable oils are obtainable from hemp, and maize corn oil. An especially preferred oil by virtue of its characteristics is sunflower (seed) oil.

Further suitable oils, that can also be emollient oils, comprise alkyl or alkyl-aryl ester oils having a boiling point of above 150°C (and a melting point of below 20°C). Such ester oils include oils containing one or two alkyl groups of 12 to 24 carbon atoms length, including isopropyl myristate, isopropyl palmitate and myristyl palmitate. Other non-volatile ester oils include alkyl or aryl benzoates such $C_{12-15}$ alkyl benzoate, for example Finsolv TN™ or Finsolv Sun™.

A further class of suitable oils comprises non-volatile dimethicones, often comprising phenyl or diphenylene substitution, for example Dow Corning 200 350cps or Dow Corning 556.

A further optional component of compositions of the invention is a thickening agent, sometimes alternatively referred to as a gelling agent or gellant. Such agents increase the viscosity of or solidify the carrier oil in which the particulate AP active is typically suspended.

The thickening agent may be selected from any of those known in the art. Often, the thickening agent includes a wax. Waxes typically are considered to melt at above 40°C and particularly between 55 and 95°C. Waxes can include ester waxes, including C12 to C24 linear fatty alcohols, waxes obtained from animals or plants, often after hydrogenation, silicone elastomers and silicone waxes. The thickening agent can comprise a mixture of particulate thickening agents, a mixture of waxes or a mixture of both types of material.

The proportion of thickening agent thickening agents is often selected in the range of from 1:30 to 1:12.5 parts per part by weight of carrier oil.

The thickening agents used in compositions according to the invention, and especially stick compositions according to the invention, are preferably selected from fibre-forming non-polymeric gelling agents and waxes, optionally supplemented by particulate silica and/or an oil-soluble polymeric thickening agent.

Waxes employed herein as thickening agents are often selected from hydrocarbons, linear fatty alcohols, silicone polymers, esters of fatty acids or mixtures containing such compounds along with a minority (less than 50% w/w and often less than 20% w/w) of other compounds. Naturally occurring waxes are often mixtures of compounds which include a substantial proportion of fatty esters.

Examples of hydrocarbon waxes include paraffin wax, ozakerite, microcrystalline wax and polyethylene wax, the last named desirably having an average molecular weight of from 300 to 600 and advantageously from 350 to 525.

Linear fatty alcohols commonly contain from 14 to 40 carbon atoms and often from 16 to 24. Preferred thickening agents of this class are stearyl alcohol and behenyl alcohol, with stearyl alcohol being most preferred.

Examples of ester waxes include esters of $C_{16}$-$C_{22}$ fatty acids with glycerol or ethylene glycol, which can be isolated from natural products or more conveniently synthesised from the respective aliphatic alcohol and carboxylic acid.

Examples of natural waxes include beeswax, wool wax and spermaceti wax of animal origin, and caster wax, jojoba wax, carnauba wax and candelilla wax which are of vegetable origin. Montan wax, which is an example of mineral wax, includes non-glyceride esters of carboxylic acids, hydrocarbons and other constituents.

Further waxes employable herein comprise silicone polymer waxes, including waxes which satisfy the empirical formula:

$$R\text{-}(SiMe_2\text{-}O\text{-})_x\text{-}SiMe_2R$$

in which x is at least 10, preferably 10 to 50 and R represents an alkyl group containing at least 20 carbons, preferably 25 to 40 carbons, and particularly having an average linear chain length of at least 30 carbons.

Other silicone waxes comprise copolymers of dimethicone and alkyloxymethicone, satisfying the general formula:-

$$Y\text{-}(SiMe_2\text{-}O\text{-})_y(Si[OR']Me\text{-}O\text{-})_z\text{-}Y'$$

in which Y represents $SiMe_2$-O, Y' $SiMe_2$, R' an alkyl of at least 15 carbons preferably 18 to 22 such as stearyl, y and z are both integers, totalling preferably from 10 to 50.

Waxes useful in the present invention will generally be those found to thicken cyclomethicone, when dissolved therein at a concentration of 5 to 15% by weight.

Fibre-forming thickening agents are dissolved in the carrier oil at elevated temperature and on cooling precipitate out to form a network of very thin strands that structure, i.e. thicken, the carrier oil. One particularly effective category comprises N-acyl aminoacid amides and in particular linear and branched N-acyl glutamic acid dialkylamides, such as in particular N-lauroyl glutamic acid di n-butylamide and N-ethylhexanoyl glutamic acid di n-butylamide and especially mixtures thereof. Such amido gellants can be employed in anhydrous compositions according to the present invention, if desired, with 12-hydroxystearic acid.

Other amido SMGAs include 12-hydroxystearic acid amides, and amide derivatives of di and tribasic carboxylic acids as set forth in WO 98/27954, including notably alkyl N,N'dialkyl succinamides.

Further suitable amido-containing thickening agents are described in US6410003, US7332153, US6410001, US6321841, and US6248312.

The thickening agent is typically employed in the composition at a concentration of from 1.5 to 30%. When a fibre-forming thickening agent is employed, its concentration is typically in the range of from 1.5 to 15%. When a wax is employed, its concentration is usually selected in the range of from 10 to 30%, and particularly from 12 to 24% w/w. Some highly desirable compositions comprise in combination a first thickening agent with a second thickening agent.

One category of oil-soluble polymer thickening agent which has been found suitable is a polysaccharide esterified with a mono-carboxylic acid containing at least 12 carbon atoms, and preferably a dextrin fatty acid ester such as dextrin palmitate or dextrin stearate. Commercial products are available under the trademark Rheopearl.

A second category of polymer thickening agent comprises polyamides for example those discussed in US 5500209 or US 6353076.

A third category of thickening agent comprises block copolymers of styrene with ethylene propylene and/or butylene available under the trade name KRATON, and particularly styrene ethylene/butylene styrene linear block copolymers. A related category of thickening polymer comprises polymers of alpha methylstyrene and styrene, such as those under the trade name KRISTALEX, e.g. KRISTALEX F85 having a mean molecular weight of approximately 1200. Yet another thickening polymer comprises alkyl substituted galactomannan available under the trade name N-HANCE AG.

A still further class of thickening polymers co-polymers of vinyl pyrrolidone with polyethylene containing at least 25 methylene units, such as triacontanyl polyvinylpyrrolidone, under the trade name Antaron WP-660.

Such thickening polymer is often employed in a weight ratio to the oil blend that is selected in the range of from 1:30 to 1:5, taking into account the hardness of the soft solid that is desired, the inherent ability of the chosen polymer to increase viscosity and the presence or otherwise of an additional thickening agent.

A further class of material which is well suited to forming or contributing to the formation of soft solid compositions comprises silicone elastomers. Commonly, the elastomer is non-emulsifying and most is a dimethicone/vinyldimethicone cross polymer. Such materials commonly supplied as a dispersion of the active material in cyclomethicone fluid or a non-volatile oil, typically at a concentration in the region of 10 to 20% by weight. Such elastomers are desirably present at a concentration of from 1 to 10% by weight of the composition.

A thickening agent especially well-suited to forming or contributing to the formation of a soft solid composition comprises particulate silica and especially fumed silica. It is desirable to include at least 2% and especially at least 2.5% by weight of the silica in the composition, such as in the range of up to 10% by weight.

Other components that may be included in compositions according to the invention including those described in the following paragraphs.

Wash-off agents may be included, often in an amount of up to 10%, to assist in the removal of the formulation from skin or clothing. Such wash-off agents are typically nonionic surfactants such as esters or ethers containing a $C_8$ to $C_{22}$ alkyl moiety and a hydrophilic moiety comprising a polyoxyalkylene group (POE or POP).

Skin feel improvers (e.g. talc or finely divided high molecular weight polyethylene), may be included, typically in an amount from 1 up to 10%.

Skin moisturisers, such as glycerol or polyethylene glycol (e.g. mol. wt. 200 to 600) may be included, typically in an amount of up to 5%.

Skin benefit agents, such as allantoin or lipids, may be included, typically in an amount of up to 5%.

A highly preferred optional component is a preservative, such as ethyl or methyl parabens or BHT (butyl hydroxy toluene), typically in an amount of from 0.01 to 0.1%.

Other components that may be present include short chain ($C_2$-$C_4$) alcohols and especially polyols such glycerol, ethylene glycol, propylene glycol and polymers thereof, in particular poly(ethylene glycol) and polypropylene glycol). Poly(ethylene glycol) of average molecular weight 200 to 600 is a preferred component. Such components may add to the sensory properties of the composition and, when included, are typically present at from 0.5 to 10% of the total composition.

Examples

The commercially available Al-Zr AP actives indicated in Table 1 were used to prepare the following Examples and Comparative Examples. Ratios provided in the supplier's certificates of analysis were used to calculate average molecular weights and hence estimate the molar amounts used in their manufacture, as indicated in Table 1. Each active was supplied and used as an aqueous solution with the indicated levels of anhydrous solids.

Table 1

| AP Salt | Av. Mol. Wt. (g.mol$^{-1}$) | Al:Zr Ratio | Metal: Cl Ratio | Glycine (%w/w) | Anh. solids (%w/w) |
|---|---|---|---|---|---|
| Octa | 948.8 | 8.41 | 1.30 | 3.3 | 32.0 |
| Penta | 761.7 | 6.70 | 1.65 | 0.0 | 36.4 |

(continued)

| AP Salt | Av. Mol. Wt. (g.mol$^{-1}$) | Al:Zr Ratio | Metal: Cl Ratio | Glycine (%w/w) | Anh. solids (%w/w) |
|---|---|---|---|---|---|
| Tetra | 494.4 | 3.54 | 1.42 | 4.3 | 27.8 |
| Tri | 680.2 | 5.69 | 1.60 | 4.6 | 28.0 |

The AP solutions indicated in Table 1 were used to prepare activated Al-Zr AP species according to Tables 2 to 5, using the following procedure.

Into 50 ml polypropylene tubes were added: deionised water (0 to 4 g, 0 to 222 mmol) and AP solution (14 to 19 g), followed by calcium chloride dihydrate (1 to 3 g, 6.8 to 20.4 mmol) and glycine as required. The weights of the components always totalled 20 g. The tubes were then capped before being shaken to observe full dissolution of the solids. The tubes were placed in racks (8 per rack), which were each placed in a water bath and heated to 87°C. The tubes were left for 2 hours at 87°C, with shaking of each tube individually at 10-minute intervals. After the two hours the racks were removed from the water bath and allowed to cool to room temperature.

HPLC analysis of each of the species produced was performed by the following method and the content of "Band 3" material is indicated in the final column of Tables 2-5 (average values and standard errors indicated are based on 3 measurements).

SEC-HPLC chromatograms were recorded using a Dionex Ultimate 3000 HPLC system and Shodex RI-101 detector using two 5$\mu$m silica porous columns in series and the chromeleon software. The program was set to inject 20 $\mu$l of sample and record the refractive index (RI) detector response over 10 minutes. The mobile phase of the column was a mixture of 0.01 M $HNO_3$ and 0.1 M NaNOs at a flow rate of 0.75 ml.min$^{-1}$. Liquid samples were made up using 0.5 g of liquid and diluted to 10 g with 0.01 M $HNO_3$. Solid samples were made up using 0.2 g of solid and diluted up to 10 g with 0.01 M $HNO_3$. Data were recorded 24 hours after the solutions were manufactured and at 25 °C.

Table 2: Octa Salts

| Sample ID | AP soln. (% w/w) | CaCl$_2$ (% w/w) | Additional Glycine (%w/w) | Additional Water (% w/w) | Band 3 HPLC (%) |
|---|---|---|---|---|---|
| Octa 1 | 83.2 | 7.8 | 2.4 | 6.6 | (40.4 ± 0.6) |
| Octa 2 | 87.0 | 8.1 | 2.0 | 2.9 | (39.2 ± 0.8) |
| Octa 3 | 78.2 | 7.3 | 1.9 | 12.6 | (45.1 ± 0.2) |
| Octa 4 | 75.7 | 7.5 | 1.9 | 14.9 | (43.5 ± 0.5) |
| Octa 5 | 77.0 | 7.1 | 2.0 | 13.9 | (42.4 ± 0.9) |
| Octa 6 | 85.6 | 6.5 | 1.9 | 6.0 | (32.6 ± 0.3) |
| Octa 7 | 80.0 | 10.1 | 1.9 | 8.0 | (42.5 ± 0.6) |
| Octa 8 | 71.6 | 9.5 | 1.9 | 17.0 | (50.1 ± 0.5) |
| Octa 9 | 85.0 | 11.6 | 2.1 | 1.7 | (43.0 ± 0.3) |
| Octa 10 | 90.0 | 5.0 | 2.0 | 2.9 | (39.1 ± 0.8) |
| Octa 11 | 85.2 | 8.9 | 2.5 | 3.4 | (46.4 ± 0.8) |
| Octa 12 | 78.1 | 8.4 | 1.5 | 12.0 | (40.8 ± 0.2) |
| Octa 13 | 78.1 | 9.1 | 2.0 | 10.3 | (52.3 ± 0.7) |
| Octa 14 | 83.0 | 8.9 | 2.1 | 6.1 | (43.9 ± 0.7) |

Table 3: Tri Salts

| Sample ID | AP soln. (% w/w) | CaCl$_2$ (% w/w) | Additional Glycine (%w/w) | Additional Water (% w/w) | Band 3 HPLC (%) |
|---|---|---|---|---|---|
| Tri 1 | 75.0 | 7.4 | 2.4 | 15.2 | (63.6 ± 0.6) |
| Tri 2 | 84.4 | 9.3 | 2.2 | 4.1 | (59.0 ± 0.9) |
| Tri 3 | 82.4 | 9.4 | 1.8 | 6.4 | (57.5 ± 0.5) |
| Tri 4 | 72.0 | 8.1 | 1.7 | 18.2 | (59.8 ± 0.6) |
| Tri 5 | 88.4 | 5.7 | 2.2 | 3.7 | (50.6 ± 0.3) |
| Tri 6 | 76.3 | 12.0 | 2.6 | 9.1 | (60.1 ± 0.8) |
| Tri 7 | 78.5 | 9.6 | 1.2 | 10.6 | (52.0 ± 0.9) |
| Tri 8 | 74.5 | 9.4 | 2.2 | 13.9 | (66 ± 1) |
| Tri 9 | 79.8 | 7.2 | 1.9 | 11.1 | (56.7 ± 0.7) |

(continued)

| Sample ID | AP soln. (% w/w) | CaCl$_2$ (% w/w) | Additional Glycine (%w/w) | Additional Water (% w/w) | Band 3 HPLC (%) |
|---|---|---|---|---|---|
| Tri 10 | 86.1 | 7.4 | 2.1 | 11.4 | (55.0 ± 0.7) |
| Tri 11 | 76.3 | 8.8 | 2.0 | 12.9 | (64.8 ± 0.4) |
| Tri 12 | 78.2 | 9.9 | 1.9 | 10.0 | (60.1 ± 0.8) |
| Tri 13 | 73.0 | 7.6 | 2.2 | 17.2 | (65.2 ± 0.5) |
| Tri 14 | 81.9 | 7.1 | 2.3 | 8.7 | (57.7 ± 0.3) |

Table 4: Tetra Salts

| Sample ID | AP soln. (% w/w) | CaCl$_2$ (% w/w) | Additional Glycine (%w/w) | Additional Water (% w/w) | Band 3 HPLC (%) |
|---|---|---|---|---|---|
| Tetra 1 | 83.1 | 12.5 | 3.0 | 1.4 | (75.5 ± 0.5) |
| Tetra 2 | 87.5 | 8.7 | 2.0 | 1.8 | (73.0 ± 0.5) |
| Tetra 3 | 75.0 | 9.8 | 2.7 | 12.5 | (75.2 ± 0.7) |
| Tetra 4 | 78.1 | 8.5 | 1.3 | 12.1 | (70.4 ± 0.4) |
| Tetra 5 | 80.4 | 8.1 | 1.4 | 10.1 | (68.8 ± 0.4) |
| Tetra 6 | 82.2 | 7.6 | 1.7 | 8.5 | (73.0 ± 0.5) |
| Tetra 7 | 84.1 | 7.0 | 2.1 | 6.8 | (76.0 ± 0.8) |
| Tetra 8 | 90.5 | 6.5 | 1.5 | 1.5 | (67.0 ± 0.2) |
| Tetra 9 | 77.6 | 10.2 | 1.8 | 10.4 | (72.8 ± 0.6) |
| Tetra 10 | 77.6 | 10.2 | 2.2 | 10.0 | (74.7 ± 0.3) |
| Tetra 11 | 72.8 | 10.5 | 1.7 | 15.0 | (73.8 ± 0.8) |
| Tetra 12 | 85.9 | 10.2 | 1.9 | 2.0 | (74.9 ± 0.5) |
| Tetra 13 | 88.1 | 9.0 | 2.3 | 0.6 | (74.2 ± 0.8) |
| Tetra 14 | 92.3 | 5.1 | 2.5 | 0.1 | (73.1 ± 0.8) |

Table 5:Penta Salts

| Sample ID | AP soln. (% w/w) | CaCl$_2$ (% w/w) | Additional Glycine (%w/w) | Additional Water (% w/w) | Band 3 HPLC (%) |
|---|---|---|---|---|---|
| Penta 1 | 76.2 | 9.5 | 6.4 | 7.9 | (83.7 ± 0.2) |
| Penta 2 | 80.0 | 8.4 | 5.6 | 6.0 | (80.1 ± 0.7) |
| Penta 3 | 78.4 | 9.6 | 6.6 | 5.4 | (84.9 ± 0.2) |
| Penta 4 | 85.1 | 6.7 | 4.6 | 3.6 | (74.7 ± 0.1) |
| Penta 5 | 72.3 | 10.5 | 6.7 | 10.5 | (81.9 ± 0.5) |
| Penta 6 | 74.2 | 11.0 | 7.2 | 7.6 | (81.5 ± 0.8) |
| Penta 7 | 90.3 | 4.4 | 4.4 | 0.9 | (56.7 ± 0.4) |
| Penta 8 | 82.6 | 8.3 | 6.3 | 2.8 | (81.3 ± 0.6) |
| Penta 9 | 79.6 | 6.5 | 5.8 | 8.1 | (80.2 ± 0.6) |
| Penta 10 | 87.5 | 5.7 | 4.4 | 2.4 | (60.4 ± 0.1) |
| Penta 11 | 81.4 | 8.6 | 6.1 | 3.9 | (80.0 ± 0.2) |
| Penta 12 | 82.9 | 9.5 | 5.1 | 2.5 | (78.2 ± 0.4) |
| Penta 13 | 77.2 | 10.7 | 7.0 | 5.1 | (81.6 ± 0.7) |
| Penta 14 | 77.9 | 10.0 | 6.5 | 5.6 | (82.4 ± 0.5) |

Reviewing Tables 2 to 5, it is clear that the highest Band 3 contents generally came from the Penta salts, followed by the Tetra salts, followed by the Tri salts, followed by the Octa salts.

In a further study, samples of each of the Octa, Penta, Tetra and Tri AP salt solutions (78.20%) were activated with the same weight percentages of calcium chloride dihydrate (7.32%), total glycine (4.45%) and additional water, using the method described above.

HPLC analysis of each of the solutions produced was performed by the method described above, producing the results indicated in Table 6.

Table 6

| Sample ID | AP soln. (% w/w) | CaCl$_2$ (% w/w) | Total Glycine (%w/w) | Additional Water (%w/w) | HPLC (%) Band 2 | Band 3 |
|---|---|---|---|---|---|---|
| Octa salt | 78.20 | 7.32 | 4.45 | To 100 | 31.25 +/- 3.0 | 53.25 +/- 1.2 |
| Penta salt | 78.20 | 7.32 | 4.45 | To 100 | 10.61 +/-2.5 | 70.26 +/-0.6 |
| Tetra salt | 78.20 | 7.32 | 4.45 | To 100 | 20.35 +/- 3.8 | 64.43 +/-2.1 |
| Tri salt | 78.20 | 7.32 | 4.45 | To 100 | 19.49 +/- 2.6 | 63.67 +/1 3.7 |

The Band 3 to Band 2 ratio was by far the highest for the Penta salt (6.6: 1) and lowest for the Octa salt (1.7: 1). The Tetra salt and Tri salt were 3.2: 1 and 3.3: 1, respectively.

In a further study, examples as indicated in Table 7 were prepared using the same activation method as described above. The levels of glycine and calcium chloride used in these examples were each expected to deliver maximum Band 3 content for each of the salts concerned, based upon computer modelling.

Table 7

| Al/Zr Salt | AP soln. (%w/w) | CaCl$_2$ (%w/w) | Total Glycine (%w/w) | Water (%w/w) | Al:Zr Ratio | M:Cl Ratio |
|---|---|---|---|---|---|---|
| Octa | 75.2 | 8.5 | 5.3 | 14.2 | 8.41 | 0.99 |
| Penta | 73.6 | 10.4 | 7.0 | 9.1 | 6.70 | 1.16 |
| Tetra | 78.3 | 10.4 | 6.6 | 9.0 | 3.54 | 0.96 |
| Tri | 74.3 | 9.5 | 6.7 | 14.1 | 5.69 | 1.05 |

HPLC analysis of each of the solutions produced was performed by the method described above, producing the results indicated in Table 8. The average values and standard errors indicated are again based on 3 measurements. Also given are the molar ratios of aluminium to calcium and aluminium to glycine for the samples.

Table 8

| Al/Zr Salt | Molar ratios Al: Ca | Al: Glycine | Band 2 / % | Band 3 / % | Band 3: Band 2 |
|---|---|---|---|---|---|
| Octa | 3.77 | 3.05 | 30.0 ± 0.4 | 55.0 ± 0.6 | 1.83 |
| Penta | 3.33 | 2.53 | 11.1 ± 0.2 | 87.2 ± 0.3 | 7.85 |
| Tetra | 2.20 | 1.77 | 19.2 ± 0.2 | 71.3 ± 0.4 | 3.72 |
| Tri | 2.69 | 1.95 | 17.6 ± 0.3 | 69.4 ± 0.2 | 3.95 |

The results obtained were very similar to those reported in Table 6, with the Band 3 to Band 2 ratio being far highest for the Penta salt and lowest for the Octa salt.

Oil-in-water emulsion roll-on compositions were prepared using the components detailed in Table 9.

Table 9

| Ingredient | Function |
|---|---|
| Deionised Water | Balancing ingredient |
| Glycerol | Humectant |
| Steareth-20 | Emulsifier |
| Disodium EDTA | Preservative |
| Al/Zr solution | AP active |
| PPG-15 Stearyl Ether | Emollient |
| Steareth-2 | Emulsifier |
| Pentaerythrityl tetra-di-t-butyl hydrocinnamate | Oxidation Stabiliser |

The following procedure was used to prepare 3 kg samples of four different emulsion compositions. Deionised water (1120 g) was added to a 3 L beaker (1) charged with a magnetic stirrer bar. Glycerol (120 g), Steareth-20 (18 g), disodium EDTA (3 g) and Al/Zr solution (balanced with water to reach 12 % (w/w of the final compositions) anhydrous Al/Zr

active (excluding $CaCl_2$ and glycine) were added. The mixture was heated to 55 °C with stirring in a water bath until no solids remained. A separate 250 mL beaker (2) was charged with a magnetic stirrer bar, PPG-15 stearyl ether (120 g), steareth-2 (78 g) and pentaerythrityl tetra-di-t-butyl hydrocinnamate (1.5 g). The second beaker was heated to 90 °C using a water bath with stirring until no solids remained. The contents of (1) were then transferred to a further vessel (3). Whilst shearing at 10,000 rpm, the contents of (2) were added to the vessel (3) over 2 minutes. The resulting emulsion was left to cool to 45 °C before being sheared at 5000 rpm. pH measurements were made after 24 hours using a Mettler Toledo SevenCompact S220 with a Mettler Toledo Inlab® Expert Pro pH electrode (calibrated using buffers at pH 4, 7 and 10. All measurements were recorded at 25 °C.

Further details of the four different oil-in-water emulsion compositions and their measured pH values are indicated in Table 10. The Al/Zr solutions used were prepared according to the method detailed above, adjusting $CaCl_2$ and glycine levels accordingly.

Table 10

| Al/Zr Salt | Al/Zr salt (anhydrous solids, % w/w) | $CaCl_2.2H_2O$ (% w/w) | Total Glycine (%w/w) | pH |
|---|---|---|---|---|
| **Octa** | 12 | 4.23 | 2.64 | 3.44 +/- 0.03 |
| **Penta** | 12 | 4.66 | 3.85 | 3.74 +/- 0.02 |
| **Tetra** | 12 | 5.72 | 3.63 | 3.61 +/- 0.04 |
| **Tri** | 12 | 5.48 | 3.87 | 3.79 +/- 0.05 |

It can be seen that the emulsion composition with the Octa salt gave a significantly lower pH value, indicating a greater potential of irritation from this composition.

The oil-in-water emulsion compositions prepared as described above and detailed in Table 10 were stored at 45°C in an oven and monitored over 12 weeks to assess rheological stability. Rheological stability was assessed in terms of the extent of (undesired) splitting of the compositions and the results are given in Table 11.

Table 11

| Al/Zr Salt | Extent of splitting (% v/v) after... | | |
|---|---|---|---|
| | 4 weeks | 8 weeks | 12 weeks |
| **Octa** | 1.92 | 9.62 | 11.54 |
| **Penta** | 0 | 4 | 4 |
| **Tetra** | 2 | 6 | 8 |
| **Tri** | 0 | 1.96 | 7.84 |

It is clear from these results that the composition with the Penta salt had least extent of splitting after 12 weeks and hence the greater long term stability. The composition with the Octa salt had the greatest extent of splitting and hence the shortest long term stability.

Stability tests were also performed on the simple aqueous solutions of the Al/Zr solutions as indicated in Table 7 and the associated description. Each of the computer-optimised AP active solutions was stored in a 25 °C oven and monitored over 12 weeks. The samples were checked periodically and any changes noted. Full gelation was observed in the Octa and Tri samples, but not in Tetra or Penta samples, indicating superior rheological stability, in terms non-gelation, for these samples.

An anhydrous stick composition as detailed in Table 12 was prepared by the following method.

In a first stage, 8808 grams of Rezal 67 (an aqueous solution of aluminium zirconium pentachlorohydrate, ca. 33.5% solids) was added to a mixing vessel, together with 469 grams of calcium chloride dihydrate and 723 grams of glycine. The mixture was heated to 85 to 90°C and held for 2 hours before it was allowed to cool to room temperature. The resulting aqueous solution was then spray dried to produce an AP powder that was used in a second stage.

In the second stage, all of the liquid ingredients and the wax were weighed into a beaker and the beaker was covered. The mixture was heated to 88 to 90°C with constant stirring. The beaker was then uncovered and the contents cooled to 78°C before the AP powder and talc were slowly added. The molten mix was then poured into stick packs at 65°C and allowed to cool to ambient temperature and solidify.

Table 12

| Component | Amount (wt. %) |
|---|---|
| Cyclopentasiloxane | 35.26 |

(continued)

| Component | Amount (wt. %) |
|---|---|
| AP powder | 23.19 |
| PPG-14 butyl ether | 17.50 |
| Stearyl alcohol | 17.50 |
| Hydrogenated castor oil (MP80) | 2.50 |
| PEG-8 distearate | 2.00 |
| Magnesium silicate (talc) | 2.00 |
| Buytlatedhydroxytoluene | 0.05 |

## Claims

1. A method of manufacture of an aluminium-zirconium (Al/Zr) antiperspirant active comprising the heating in aqueous solution of an aluminium-zirconium penta-chlorohydrex-glycine complex and a calcium salt having a solubility in water of 10 g/L or greater measured at 20°C and 1 atmosphere pressure, the molar ratio of aluminium to calcium being from 1.3 to 60.

2. A method of manufacture according to claim 1, wherein the molar ratio of aluminium to glycine is from 1.5 to 25.

3. A method of manufacture according to claim 1 or claim 2, wherein the aqueous solution has a pH of from 3.5 to 4.1.

4. A method of manufacture according to any one of the preceding claims, wherein the aluminium-zirconium penta-chlorohydrex-glycine complex is formed *in situ* from an aluminium-zirconium penta-chlorohydrex salt and glycine.

5. A method according to any one of preceding claims, wherein the molar ratio of aluminium to calcium is from 1.8 to 20.

6. A method according to any one of the preceding claims, wherein the aqueous solution is heated to 50°C or above for 30 minutes or longer.

7. A method according to any of claims 2 to 6, wherein the molar ratio of aluminium to glycine is from 1.75 to 1: 10.

8. A method according to any one of the preceding claims, wherein the resulting aqueous solution is subsequently spray-dried to give a solid particulate AP active.

9. A method according to claim 8, wherein the solid particulate AP active is subsequently mixed with a carrier oil and structuring agent to form a solid stick or soft solid composition.

10. A method according to any one of the preceding claims, wherein the calcium salt is calcium chloride.

11. A method according to any one of the preceding claims, wherein the resulting Al/Zr AP active is subsequently mixed with a fragrance or an emollient oil.

12. A method according to any one of claims 10 or 11, wherein the resulting Al/Zr AP active in aqueous solution is emulsified with a fragrance and/or an emollient oil with shear and an emulsifier.

13. An AP composition manufactured by a method according to any one of the preceding claims.

14. A non-therapeutic method of reducing perspiration from the surface of the human body, comprising the topical application of a composition according to claim 13.

**Patentansprüche**

1.  Verfahren zur Herstellung eines transpirationshemmenden Aluminium-Zirconium (Al/Zr)-Wirkstoffs, umfassend das Erhitzen eines Aluminium-Zirconium-Pentachlorhydrex-Glycin-Komplexes und eines Calciumsalzes, das eine Löslichkeit in Wasser von 10 g/l oder mehr, gemessen bei 20°C und einem Druck von einer Atmosphäre, aufweist, wobei das Molverhältnis von Aluminium zu Calcium 1,3 bis 60 beträgt.

2.  Verfahren zur Herstellung nach Anspruch 1, wobei das Molverhältnis von Aluminium zu Glycin 1,5 bis 25 beträgt.

3.  Verfahren zur Herstellung nach Anspruch 1 oder Anspruch 2, wobei die wässrige Lösung einen pH-Wert von 3,5 bis 4,1 aufweist.

4.  Verfahren zur Herstellung nach irgendeinem der vorhergehenden Ansprüche, wobei der Aluminium-Zirconium-Pentachlorhydrex-Glycin-Komplex in situ aus einem Aluminium-Zirconium-Pentachlorhydrex-Salz und Glycin gebildet wird.

5.  Verfahren nach irgendeinem der vorhergehenden Ansprüche, wobei das Molverhältnis von Aluminium zu Calcium 1,8 bis 20 beträgt.

6.  Verfahren nach irgendeinem der vorhergehenden Ansprüche, wobei die wässrige Lösung 30 Minuten oder länger auf 50°C oder mehr erhitzt wird.

7.  Verfahren nach einem der Ansprüche 2 bis 6, wobei das Molverhältnis von Aluminium zu Glycin 1,75 bis 1:10 beträgt.

8.  Verfahren nach irgendeinem der vorhergehenden Ansprüche, wobei die anfallende wässrige Lösung anschließend sprühgetrocknet wird, um einen festen teilchenförmigen transpirationshemmenden Wirkstoff zu erhalten.

9.  Verfahren nach Anspruch 8, wobei der feste teilchenförmige transpirationshemmende Wirkstoff anschließend mit einem Trägeröl und einem Strukturierungsmittel vermischt wird, um einen festen Stab oder eine weiche Feststoffzusammensetzung zu bilden

10. Verfahren nach irgendeinem der vorhergehenden Ansprüche, wobei das Calciumsalz Calciumchlorid ist.

11. Verfahren nach irgendeinem der vorhergehenden Ansprüche, wobei der erhaltene transpirationshemmende Al/Zr-Wirkstoff anschließend mit einem Duftstoff oder einem Weichmacheröl vermischt wird.

12. Verfahren nach einem der Ansprüche 10 oder 11, wobei der erhaltene transpirationshemmende Al/Zr-Wirkstoff in wässriger Lösung mit einem Duftstoff und/oder einem Weichmacheröl unter Scherung und mit einem Emulgator emulgiert wird.

13. Transpirationshemmende Zusammensetzung, hergestellt nach dem Verfahren gemäß irgendeinem der vorhergehenden Ansprüche.

14. Nicht-therapeutisches Verfahren zur Verringerung der Schweißbildung auf der Oberfläche des menschlichen Körpers, umfassend die topische Verwendung einer Zusammensetzung gemäß Anspruch 13.


**Revendications**

1.  Procédé de fabrication d'un actif antitranspirant d'aluminium-zirconium (Al/Zr) comprenant le chauffage en solution aqueuse d'un complexe penta-chlorohydrex d'aluminium et de zirconium-glycine et d'un sel de calcium ayant une solubilité dans l'eau de 10 g/L ou plus mesurée à 20°C et à une pression de 1 atmosphère, le rapport molaire de l'aluminium au calcium étant de 1,3 à 60.

2.  Procédé de fabrication selon la revendication 1, dans lequel le rapport molaire de l'aluminium à la glycine est de 1,5 à 25.

3.  Procédé de fabrication selon la revendication 1 ou la revendication 2, dans lequel la solution aqueuse a un pH de 3,5 à 4,1.

4. Procédé de fabrication selon l'une quelconque des revendications précédentes, dans lequel le complexe penta-chlorohydrex d'aluminium et de zirconium-glycine est formé *in situ* à partir d'un sel de penta-chlorohydrex d'aluminium et de zirconium et de glycine.

5. Procédé selon l'une quelconque des revendications précédentes, dans lequel le rapport molaire de l'aluminium au calcium est de 1,8 à 20.

6. Procédé selon l'une quelconque des revendications précédentes, dans lequel la solution aqueuse est chauffée à 50°C ou plus pendant 30 minutes ou plus.

7. Procédé selon l'une quelconque des revendications 2 à 6, dans lequel le rapport molaire de l'aluminium à la glycine est de 1,75 à 1:10.

8. Procédé selon l'une quelconque des revendications précédentes, dans lequel la solution aqueuse résultante est ensuite séchée par pulvérisation pour donner un actif AT particulaire solide.

9. Procédé selon la revendication 8, dans lequel l'actif AT particulaire solide est ensuite mélangé à une huile de support et à un agent structurant pour former un bâtonnet solide ou une composition solide molle.

10. Procédé selon l'une quelconque des revendications précédentes, dans lequel le sel de calcium est le chlorure de calcium.

11. Procédé selon l'une quelconque des revendications précédentes, dans lequel l'actif AT Al/Zr résultant est ensuite mélangé avec un parfum ou une huile émolliente.

12. Procédé selon l'une quelconque des revendications 10 ou 11, dans lequel l'actif AT Al/Zr en solution aqueuse résultant est émulsionné avec un parfum et/ou une huile émolliente avec cisaillement et un émulsifiant.

13. Composition AT fabriquée par un procédé selon l'une quelconque des revendications précédentes.

14. Procédé non thérapeutique de réduction de la transpiration de la surface du corps humain, comprenant l'application topique d'une composition selon la revendication 13.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4359456 A, Gosling
- EP 1104282 A
- US 6911195 B
- US 5955065 A
- US 6942850 B
- WO 2009044381 P
- US 7704531 B
- US 20110038823 A
- US 2007196303 A
- US 20070020211 A
- WO 2008063188 A
- US 20080131354 A
- US 7087220 B, Summit and Reheis)
- US 20070003499 A
- EP 1104282 B1
- WO 9827954 A
- US 6410003 B
- US 7332153 B
- US 6410001 B
- US 6321841 B
- US 6248312 B
- US 5500209 A
- US 6353076 B